# EUROPÄISCHE PATENTSCHRIFT

(11) **EP 1 061 369 B1**
(45) Veröffentlichungstag und Bekanntmachung des Hinweises auf die Patenterteilung: **19.07.2006**
(21) Anmeldenummer: 00112546.7
(22) Anmeldetag: 14.06.2000
(51) Int. Cl.: G01N 33/558, G01N 33/58

(54) **Element, Verfahren und Kit zur Bestimmung eines Analyts in einer Flüssigkeit**
Element, method and kit for the determination of an analyte in a fluid
Elément, methode et trousse pour la détermination d'un analyte dans un fluide

(30) Priorität: 18.06.1999 DE 19927783
(43) Veröffentlichungstag der Anmeldung: 20.12.2000
(73) Patentinhaber: Roche Diagnostics GmbH, 68305 Mannheim (DE)
(72) Erfinder: Goerlach-Graw, Ada, Dr., 67229 Grosskarlbach (DE); Schlipfenbacher, Reiner, Dr., 67098 Bad Duerkheim (DE)

(56) Entgegenhaltungen:
- EP-A- 0 267 521
- EP-A- 0 585 912
- EP-A- 0 699 906
- EP-A- 0 926 498
- DE-A- 19 838 802
- GB-A- 2 342 992
- US-A- 5 516 635
- SCHLIPFENBACHER R L ET AL: "IMMUNOMETRIC DIPSTICK FOR RAPID AND SIMPLE SCREENING OF MICROALBUMINURIC PATIENTS" CLINICAL CHEMISTRY, Bd. 36, Nr. 6, 1990, Seite 998 XP002188970 ISSN: 0009-9147
- KATERKAMP: "Chemical and biochemical sensors" 15. Januar 2001 (2001-01-15), WILEY-VHC VERLAG GMBH & CO KGAA , GERMANY Gefunden im Internet: URL:www.mrw.interscience.wiley.com/ueic/ar ticles/b01_121/sect3.html> Ullman's Encyclopedia of Industrial Chemistry
- ROEMPP: "Affinitätschromatographie" 2003, GEORG THIEME VERLAG , GERMANY Gefunden im Internet: URL:www.roempp.com/prod/roempp.php> Roempp Lexikon der Chemie

## Beschreibung

Die Erfindung betrifft ein Element zur Bestimmung eines Analyts in einer Flüssigkeit mittels einer spezifischen Bindungsreaktion zweier bioaffiner Bindungspartner enthaltend in oder auf Material, das einen Flüssigkeitstransport zwischen Zonen ermöglicht, eine Probenauftragszone und eine hierzu flußabwärts gelegene Nachweiszone sowie zwischen Probenauftragszone und Nachweiszone eine Zone mit immobilisiertem Analyt oder Analytanalogon und in der Probenauftragszone oder flußaufwärts oder flußabwärts von ihr durch Flüssigkeit ablösbar imprägniertes Konjugat aus einem mit dem zu bestimmenden Analyt zu einer spezifischen Bindungsreaktion fähigen bioaffinen Bindungspartner und einer detektierbaren Markierung.

Solche Elemente sind beispielsweise aus DE-A3842702 oder DE-A4439429 bekannt. Die dortigen Analysenelemente enthalten die zur Durchführung immunenzymometrischer bzw. immunenzymometrisch-analoger Bestimmungsverfahren notwendigen Reagenzien. Dies sind insbesondere in einer Zone zwischen Probenauftragszone und Nachweiszone immobilisierter Analyt oder Analytanalogon und ein Konjugat aus einem mit dem zu bestimmenden Analyt zu einer spezifischen Bindungsreaktion fähigen bioaffinen Bindungspartner und einer detektierbaren Markierung.

Im Falle von DE-A 3842702 ist als detektierbare Markierung eine Enzymmarkierung beschrieben. Um diese Markierung sichtbar zu machen, ist es notwendig, sie mit einem chromogenen Enzymsubstrat in Kontakt zu bringen, so daß sich aufgrund der Enzymaktivität eine Farbe bildet. Das Erfordernis, die Markierung sichtbar zu machen, ist umständlich, aufgrund der zu treffenden Maßnahmen kostspielig und außerdem gegebenenfalls mit technischen Schwierigkeiten behaftet, beispielsweise dann, wenn das entsprechende Enzymsubstrat in dem Analysenelement Stabilitätsprobleme aufweist

In neuerer Zeit werden deshalb Direktmarkierungen bevorzugt, wie sie in DE-A 4439429 beschrieben werden. Diese Direktmarkierungen sind beispielsweise Metall- oder Latexpartikel, die von sich aus gefärbt sind und mit bloßem Auge auszumachen sind. Besonders bevorzugt wird heutzutage eine Goldmarkierung. Hierzu wird je nach Analyt ein entsprechender markierter bioaffiner Bindungspartner hergestellt, für den dann auf dem Analysenelement optimale Bedingungen für Reaktion und Lagerung geschaffen werden müssen. Diese jeweilige Anpassung an den zu bestimmenden Analyt erfordert einen hohen Aufwand. Je nach dem zu bestimmenden Analyt können sich die hierfür erforderlichen polyklonalen oder monoklonalen Antikörper sehr verschieden bei der Konjugierung an Goldpartikel verhalten. Dies kann zu unterschiedlichen Stabilitäten von Goldkonjugaten führen. Aus dem unterschiedlichen Verhalten verschiedener polyklonaler Antikörper bzw. verschiedener monoklonaler Antikörper bei der Beladung von Goldpartikeln können sich ganz unterschiedliche räumliche Anordnungen der Antikörper auf den Goldpartikeln ergeben, was zu sterischen Problemen bei der Reaktion solcher Konjugate mit Analyt führen und damit zu einer schlechten Empfindlichkeit Anlaß geben kann.

US 5,516,635 beschreibt Analyseverfahren, welche zwei verschiedene Analytbindungspartner besitzen: ein auf einer Oberfläche fixiertes capture binding agent, welches den Analyten bindet und ein developing binding agent, welches entweder an den gebundenen Analyten oder das capture binding agent bindet und zur Detektion an markierte Mikrosphären gebunden ist. Die Mikrosphären dienen nicht selbst zur Detektion, sondern als Träger der eigentlichen detektierbaren Marker. US 5,516,635 beschreibt unter anderem ein Nachweissystem, bei welchem die die Marker tragenden Mikrosphären mit Avidin markiert sind und das developing binding agent eine Biotin-Markierung trägt. Zur Detektion des Analyten werden hier jedoch erst nach Ablauf der immunologischen Reaktionen diese avidin-markierten Mikrosphären zugegeben und die Bindung dieser Mikrosphären an den Analyt-developing binding agent-Komplex erfolgt erst in einem späteren Arbeitsschritt. US 5,516,635 beschreibt weiterhin ein Nachweissystem, bei welchem eine analytspezifische Nukleinsäure mit einer Digoxigenin-Markierung verbunden ist, welche mit dem Analyten reagiert. Nach einem Waschschritt wird zur Detektion ein Konjugat aus gegen Digoxigenin gerichtetem Antikörper und Mikrosphären, welche wiederum die zur Detektion genutzten Labelmoleküle tragen, zugegeben.

EP 0 585 912 beschreibt eine Vorrichtung zur Durchführung eines Immunoassays zur Detektion von Antikörpern, bei welcher folgende drei Zonen auf einem Trägermaterial aufgebracht sind: eine Zone mit Biotin-markiertem Antigen als Analytbindepartner (biotinylated antigen zone), eine davon getrennte Zone mit an Anti-Biotin gekoppelten Detektorpartikeln (detector zone) und eine Zone mit an eine Matrix gebundenen Antigenen zur Bindung des Antikörper-Antigen-Detektorpartikel-Komplexes (capture zone). Weiterhin beschreibt EP0585912 diesbezügliche Verfahren, bei welchen die Probe mit dem Analyten zunächst auf die Vorrichtung aufgebracht wird, anschließend nacheinander die biotinylated antigen zone und die detector zone durchwandert, wobei Nachweiskomplex gebildet wird, welcher schließlich in der capture zone an die Matrix gebunden wird und dort im immobiliserten Zustand detektiert werden kann.

EP 0 267 521 beschreibt ein Verfahren zum Nachweis einer Nukleinsäurehybridisierung mittels einer an eine Matrix gebunden Nukleinsäure, welche eine zweite komplementäre Nukleinsäure, die sich in der Probenlösung befindet, bindet. Der Nachweis der Hybridisierung erfolgt über ein direkt an der zweiten Nukleinsäure gebundenes Label, welches in einem vorherigen Verfahrensschritt an die zweite Nukleinsäure gebunden wird und welches durch eine einzige Substanz, ein Konjugat aus einem Bindungspartner für das direkt an den Analyten gebundenen Label und einer detektierbaren Markierung, nachgewiesen werden kann.

Aufgabe der vorliegenden Erfindung war es deshalb, diese Nachteile zu vermeiden und ein Analysenelement bereitzustellen, das stabile, einfach und reproduzierbar herstellbare Reagenzien enthält, die eine empfindliche Bestimmungsreaktion ermöglichen.

Diese Aufgabe wird durch den Gegenstand der Erfindung gelöst, wie er in den Ansprüchen dargestellt ist.

Gegenstand der Erfindung ist ein Element zur Bestimmung eines Analyts in einer Flüssigkeit mittels einer spezifischen Bindungsreaktion zweier bioaffiner Bindungspartner wie es in Anspruch 1 definiert ist. und flußaufwärts der Zone mit immobilisiertem Analyt oder Analytanalogon ebenfalls durch Flüssigkeit ablösbares, universelles Konjugat 2 aus einem mit der detektierbaren Markierung 1 zu einer spezifischen Bindungsreaktion fähigen bioaffinen Bindungspartner 2 und einer visuell erkennbaren Markierung 2 vorhanden ist.

Gegenstand der Erfindung ist außerdem die Verwendung eines erfindungsgemäßen Elementes zur Bestimmung eines Analyts sowie ein entsprechendes Bestimmungsverfahren. Dieses Verfahren zur Bestimmung eines Analyts mittels eines erfindungsgemäßen Elementes ist dadurch gekennzeichnet, daß
die Probenauftragszone mit Analyt in Kontakt gebracht wird,
der Analyt mit Flüssigkeit in Richtung Nachweiszone bewegt wird,
in dieser Flüssigkeit befindlicher Analyt mit Konjugaten 1 und 2 zu einem Nachweiskomplex reagiert,
der Nachweiskomplex mit Flüssigkeit in die Nachweiszone transportiert
und dort bestimmt wird.

Schließlich ist Gegenstand der Erfindung ein Kit zur Bestimmung eines Analyts enthaltend ein erfindungsgemäßes Analysenelement und Elutionsmittel.

Die Bestimmung eines Analyts mittels eines erfindungsgemaßen Elementes erfolgt aufgrund spezifischer Bindungsreaktionen zweier bioaffiner Bindungspartner. Bioaffine Bindungspartner und entsprechende spezifische Bindungsreaktionen zwischen den Bindungspartnern sind dem Fachmann bekannt. Bioaffine Bindungspartner sind beispielsweise Hapten und Antikörper, Antigen und Antikörper, Lektin und Zucker oder Saccharid, Avidin oder Streptavidin und Biotin sowie Nukleinsäure und Nukleinsäure, Ligand und Rezeptor. Ein Antigen kann hierbei jedes Molekül sein, gegen das man experimentell in der Lage ist, Antikörper zu erzeugen. Auch ein Antikörper oder eine bestimmte Stelle eines Antikörpers, die als Epitop bezeichnet wird, kann ein Antigen sein und von einem Antikörper spezifisch erkannt und gebunden werden. Unter Nukleinsäuren sollen alle möglichen Formen von Nukleinsäuren verstanden werden, die in der Lage sind, Bindungen über komplementäre Basen einzugehen. Speziell. aber nicht als abschließende Liste, seien DNA, RNA, aber auch Nukleinsäureanaloga, wie Peptidnukleinsäuren (PNA, siehe beispielsweise in WO92/20702), genannt. Ligand und Rezeptor bezeichnen ganz allgemein eine spezifische Bindungswechselwirkung zwischen zwei Partnern, wie beispielsweise zwischen Hormon und Hormonrezeptor.

In dem erfindungsgemäßen Element befinden sich die für die Durchführung der Bestimmung eines Analyts notwendigen Reagenzien sowie weitere für die Funktion des Elementes notwendige Zonen in oder auf Material, das einen Flüssigkeitstransport ermöglicht. Wesentlich für das erfindungsgemäße Analysenelement ist, daß sich Flüssigkeit innerhalb des Elementes in Richtung auf die Nachweiszone bewegen kann. Ein solcher Flüssigkeitsfluß ist beispielsweise in einem entsprechend hergerichteten Hohlkörper durch Schwerkraft möglich. Vorrichtungen, die einen Flüssigkeitstransport durch Zentrifugalkraft als eine Form der Schwerkraft ermöglichen, sind beispielsweise aus EP-B 0052769 bekannt. Bevorzugt enthalten erfindungsgemäße Analysenelemente jedoch saugfähige Materialien, die Flüssigkeit durch Kapillarkraft zu bewegen vermögen. Die Materialien der einzelnen Zonen des erfindungsgemäßen Elementes können hierbei gleich oder auch verschieden sein. Häufig wird es so sein, daß unterschiedliche Zonen aus unterschiedlichen Materialien bestehen, wenn diese optimal ihre Aufgabe erfüllen sollen.

Als mögliche saugfähige kapillaraktive Materialien kommen grundsätzlich alle solche in Frage, die generell in sogenannten "Trockentesten", wie sie beispielsweise in US-A 4,861,711, US-A 5,591,645 oder EP-A 0291194 oder in DE-A 3842702 oder DE-A 4439429 beschrieben sind, zur Flüssigkeitsaufnahme eingesetzt werden können. Als vorteilhaft haben sich hierfür beispielsweise poröse Materialien, wie Membranen, beispielsweise Nitrocellulose-Membranen, erwiesen. Es können jedoch auch faserige, saugfähige Matrixmaterialien, wie Vliese, Gewebe oder Gewirke, eingesetzt werden. Vliese sind besonders bevorzugt. Faserige Matrixmaterialien können Glas, Zellulose, Zellulosederivate, Polyester, Polyamid, aber auch Viskose, Zellwolle und Polyvinylalkohol enthalten. Vliese aus Fasern auf der Basis von Zellulose, Polymerisatfasern auf Basis von Polyester und/oder Polyamid und einem organischen Bindemittel, das OH- und/oder Estergruppen aufweist, wie sie aus EP-B 0326135 bekannt sind, sind beispielsweise erfindungsgemäß einsetzbar. Vliesmaterialien enthaltend schmelzbare Copolyesterfasern neben Glasfasern, Polyesterfasern, Polyamidfasern, Zellulosefasern oder Zellulosederivatefasern, wie sie in der Europäischen Patentanmeldung 0571941 beschrieben sind, können auch in dem erfindungsgemäßen Analysenelement eingesetzt werden. Papiere, wie beispielsweise Teebeutelpapier, sind ebenfalls gut einsetzbar.

Zur Verbesserung der Handhabung des erfindungsgemäßen Analysenelementes kann sich das saugfähige kapillaraktive Material oder können sich unterschiedliche saugfähige, kapillaraktive Materialien auf einem steifen Trägermaterial angeordnet befinden, welches seinerseits für Flüssigkeit nicht durchlässig ist, den Flüssigkeitsfluß im Matrixmaterial nicht negativ beeinflußt und sich in bezug auf die im Analysenelement ablaufenden Reaktionen inert verhält. Bevorzugtes Trägermaterial kann beispielsweise Polyesterfolie sein, auf der das den Flüssigkeitstransport ermöglichende Matrixmaterial befestigt ist.

In dem erfindungsgemäßen Element können die einzelnen Zonen übereinander, nebeneinander oder teils übereinander und teils nebeneinander auf dem Trägermaterial angeordnet sein. Besonders bevorzugt ist ein erfindungsgemäßes Analysenelement, bei dem sich Probenauftragszone, Zone mit immobilisiertem Analyt oder Analytanalogon und Nachweiszone nebeneinander auf dem Trägermaterial angeordnet befinden. Nebeneinander bedeutet in diesem Zusammenhang, daß sich diese Zonen in Richtung des Flüssigkeitstransports in direktem Kontakt miteinander befinden oder aber durch andere Zonen getrennt im wesentlichen in einer Ebene angeordnet sind.

Die Probenauftragszone ist der Bereich des erfindungsgemäßen Elementes, auf den die Probe, in der bestimmt werden soll, ob ein bestimmter Analyt anwesend, gegebenenfalls in welcher Menge er anwesend ist, aufgegeben wird.

Die Nachweiszone ist der Bereich des erfindungsgemäßen Analysenelementes, in dem die Bestimmung erfolgt, ob der untersuchte Analyt in der auf das Element aufgegebenen Probe vorhanden war. Diese Bestimmung kann qualitativ, halbquantitativ oder quantitativ sein. Halbquantitativ bedeutet hierbei, daß für den Analyt kein konkreter Konzentrationswert, sondern ein Konzentrationsbereich ermittelt wird, in dem sich die Analytkonzentration befindet.

Erfindungsgemäß befindet sich zwischen Probenauftragszone und Nachweiszone eine Zone mit immobilisiertem Analyt oder Analytanalogon. Unter Analytanalogon wird hierbei eine Substanz verstanden, die sich dem zu bestimmenden Analyten in bezug auf die spezifische Bindungsreaktion mit dem bioaffinen Bindungspartner vergleichbar verhält.

Die Immobilisierung von Analyt oder Analytanalogon auf einem Matrixmaterial zwischen Probenauftragszone und Nachweiszone kann nach dem Fachmann bekannten Methoden erfolgen. So ist es beispielsweise möglich, Analyt oder Analytanalogon auf das geeignete Matrixmaterial so zu adsorbieren, daß unter Testbedingungen eine Ablösung des Analyts oder Analytanalogons mit Flüssigkeit nicht erfolgt. Natürlich kann eine Immobilisierung aber auch chemisch unter Eingehen kovalenter Bindungen erfolgen. Ein Analyt kann entweder direkt oder über einen Spacer an das Matrixmaterial immobilisiert werden. Im Falle eines Spacers wird man in der Regel den Analyt mit einem entsprechenden Spacer chemisch modifizieren und dann dieses Analytanalogon an das Matrixmaterial binden. Eine indirekte Bindung des Analyts oder Analytanalogons an das Matrixmaterial kann aber auch über zwei bioaffine Bindungspartner, wie beispielsweise Biotin und Streptavidin erfolgen.

Als Analytanalogon haben sich für den Fall des Nachweises von Haptenen Polyhaptene als besonders geeignet erwiesen. Polyhaptene sind Substanzen, die eine Vielzahl von Haptenen aufweisen, so daß auch eine Vielzahl bioaffiner Bindungspartner spezifisch daran gebunden werden können. Aufgrund der so erzielbaren hohen Dichte an bioaffinen Bindungspartnern kann eine hohe Empfindlichkeit des Tests erzielt werden.

Erfindungsgemäß notwendig ist auf dem Element die Anwesenheit zweier Konjugate. Diese Konjugate können sich in der Probenauftragszone befinden. Sie können aber auch flußaufwärts oder flußabwärts der Probenauftragszone angeordnet sein. Die Konjugate können in das entsprechende Matrixmaterial imprägniert vorliegen. Sie können aber auch auf das Matrixmaterial beschichtet sein. Die Konjugate 1 und 2 können als Mischung vorliegen. Sie können aber auch getrennt angeordnet sein und müssen sich dann auch nicht unbedingt direkt nebeneinander befinden.In letzterem Fall ist es dann auch möglich, daß sich beispielsweise ein Konjugat flußaufwärts der Probenauftragszone befindet und das andere Konjugat beispielsweise in einer Zone zwischen Probenauftragszone und Zone mit immobilisiertem Analyt oder Analytanalogon.

Konjugat 1 besteht aus einem mit dem zu bestimmenden Analyt zu einer spezifischen Bindungsreaktion fähigen bioaffinen Bindungspartner 1 und einer detektierbaren Markierung 1. Für den Fall, daß es sich bei dem zu bestimmenden Analyt um ein Hapten oder ein Antigen handelt, wird der bioaffine Bindungspartner 1 ein Antikörper sein, der mit dem Analyt eine spezifische Bindungsreaktion eingehen kann. Für den Fall, daß es sich bei dem Analyt um einen Antikörper handelt, kann der bioaffine Bindungspartner 1 ein entsprechendes Hapten oder Antigen sein, das mit dem Antikörper eine spezifische Bindungsreaktion eingeht.

Unter der detektierbaren Markierung 1 wird erfindungsgemäß ein niedermolekulares organisches Molekül verstanden, vorzugsweise ein organisches Molekül mit einem Molekulargewicht kleiner 1.500, ganz besonders bevorzugt kleiner 1.000, das als Hapten von einem entsprechenden Antikörper in einer spezifischen Bindungsreaktion gebunden und so detektiert werden kann. Hervorragend geeignet für diesen Zweck haben sich Digoxygenin oder Digoxin erwiesen. Ganz besonders bevorzugt ist Digoxin.

Die Markierung 1 ist bevorzugt kovalent an den bioaffinen Bindungspartner 1 gebunden. Solche Konjugate sind mit einfachen organisch-chemischen Reaktionen reproduzierbar herstellbar.

Das Konjugat 2 ist ein, unabhängig von dem zu bestimmenden Analyt, universell einsetzbares Konjugat aus einem mit der detektierbaren Markierung 1 zu einer spezifischen Bindungsreaktion fähigen bioaffinen Bindungspartner 2 und einer visuell erkennbaren Markierung 2. Besonders bevorzugt ist der bioaffine Bindungspartner 2, ein Antikörper gegen das als Markierung 1 eingesetzte niedermolekulare organische Molekül. Als visuell erkennbare Markierung 2 werden bevorzugt sogenannte Direktlabel eingesetzt, d. h. Markierungen, die ohne weitere Handhabungsschritte mit dem Auge durch ihre Farbe erkennbar sind. Vorteilhafte Markierungen dieser Art sind beispielsweise in Wasser unlösliche Partikel, wie Metall- oder Latexpartikel, aber auch Pigmente wie Silikat, Ruß oder Selen. Insbesondere Metallpartikel werden erfindungsgemäß bevorzugt als Markierung eingesetzt. Kolloidales Gold ist als Markierung ganz besonders bevorzugt. Die Markierung 2 kann an den bioaffinen Bindungspartner 2 kovalent oder auch adsorptiv gebunden sein, wobei adsorptiv alle Möglichkeiten, außer kovalenter Bindung, einschließt. Bei kolloidalen Metallen als Direktmarkierungen, insbesondere bei kolloidalem Gold, werden vorzugsweise adsorptive Bindungen genutzt.

Erfindungsgemäß wesentlich ist es, daß beide Konjugate sich so in oder auf Matrixmaterial flußaufwärts der Zone mit immobilisiertem Analyt oder Analytanalogon befinden, daß sie durch Flüssigkeit ablösbar und in Richtung der Nachweiszone transportierbar sind.

Erfindungsgemäß können sich beide Konjugate oder auch nur eines der Konjugate flußaufwärts der Probenauftragszone in dem erfindungsgemäßen Element befinden. In diesem Fall ist es erforderlich, daß sich flußaufwärts der Probenauftragszone auch eine Elutionsmittelaufgabezone befindet. In diesem Fall ist es nämlich erforderlich, daß, nachdem Analyt enthaltende Probe auf die Probenauftragszone aufgegeben worden ist, mit einem extra aufgegebenen Elutionsmittel das Konjugat/die Konjugate durch die Probenauftragszone in Richtung der Nachweiszone transportiert wird/werden. Als Elutionsmittel kommen dabei Wasser oder geeignete wässrige Lösungen, wie Puffer in Frage.

In einer alternativen Ausführungsform des erfindungsgemäßen Analysenelementes ist keine von der Probenauftragszone getrennte Elutionsmittelaufgabezone vorgesehen. Dies ist dann ausreichend, wenn die Probenauftragszone flußaufwärts der Zone oder Zonen mit den Konjugaten, der Zone mit immobilisiertem Analyt oder Analytanalogon und der Nachweiszone angeordnet ist

Möglich ist es auch, daß sich flußaufwärts zur Probenauftragszone eine Elutionsmittelaufgabezone entweder auf getrennten Matrixmaterialien oder dem gleichen Matrixmaterial befindet.

Der Einsatz zweier wie vorstehend beschriebener Konjugate in einem erfindungsgemäßen Element zur Bestimmung eines Analyts besitzt erhebliche Vorteile gegenüber den Ausführungsformen des Standes der Technik. So ist das universell einsetzbare Konjugat 2 ein stabiles Konjugat aus einer visuell erkennbaren Markierung und einem bioaffinen Bindungspartner, das eine hohe Empfindlichkeit gegenüber einem niedermolekularen organischen Molekül aufweist. Konjugat 2 ist reproduzierbar herstellbar. Durch die Verwendung eines niedermolekularen organischen Moleküls als detektierbarer Markierung 1 ist es möglich, polyklonale Antikörper oder monoklonale Antikörper durch chemische Methoden einfach und in gleicher Qualität reproduzierbar zu konjugieren. Es werden so definierte Produkte hergestellt. Üblicherweise ist bei Immunoassays mit markierten Bindungspartnern des Analyts der markierte Bindungspartner eine kritische Komponente. Optimierungsarbeit, insbesondere in bezug auf die Lagerfähigkeit der Komponente auf dem Analysenelement aber auch bereits vor der Verarbeitung und der Empfindlichkeit dieser Komponente, entfällt mit der vorliegenden Erfindung weitgehend oder wird zumindest sehr erleichtert. Hier ist auch die Optimierung von Reaktionsbedingungen auf dem Analysenelement und die Eluierbarkeit des Konjugats im Analysenelement zu nennen, die so weitgehend standardisiert werden können. Diese Arbeiten beschränken sich im wesentlichen nur noch auf Konjugat 1, das aufgrund der Natur seiner Komponenten aber wesentlich einfacher zu optimieren ist als ein Konjugat aus je nach Analyt wechselndem bioaffinem Bindungspartner und visuell erkennbarer Markierung, die beide oft sehr heterogen und nicht genau bestimmt sein können und so in Bezug auf das Produkt nur schwer in gleicher, reproduzierbarer Qualität hergestellt werden können.

Die Bestimmung eines Analyts mit einem erfindungsgemäßen Element erfolgt so, daß Probe, die auf die Anwesenheit von Analyt untersucht werden soll, mit der Probenauftragszone in Kontakt gebracht wird. Entweder ist der Analyt selbst bereits in Flüssigkeit gelöst oder suspendiert oder es wird zusätzliche Flüssigkeit als Elutionsmittel auf das Element aufgegeben, um die Inhaltsstoffe der Probe, insbesondere den gegebenenfalls anwesenden Analyt, mit Flüssigkeit in Richtung der Nachweiszone zu bewegen. Der in der Probe anwesende Analyt wird hierbei mit der Mischung aus den Konjugaten 1 und 2 in Kontakt kommen und mit ihnen zu einem Nachweiskomplex reagieren. Dieser Nachweiskomplex wird mit Flüssigkeit in die Nachweiszone transportiert und dort bestimmt. Nur wenn Analyt in der Probe vorhanden war, wird visuell erkennbare Markierung 2 in Form des zuvor genannten Nachweiskomplexes in die Nachweiszone gelangen und dort detektiert werden können. Wenn kein Analyt in der untersuchten Probe war, wird die Mischung aus Konjugaten 1 und 2 in der Zone mit immobilisiertem Analyt oder Analytanalogon gebunden und es wird keine visuell erkennbare Markierung 2 in die Nachweiszone gelangen. Um optimal funktionieren zu können, werden sowohl Konjugat 1 als auch Konjugat 2 in einer solchen Konzentration vorliegen, daß der in der Zone zwischen Probenauftragszone und Nachweiszone immobilisierte Analyt oder das Analytanalogon die Konjugate vollständig zu binden vermögen. In bezug auf den zu bestimmenden Analyt sollen die Konjugate und dementsprechend auch der immobilisierte Analyt oder das Analytanalogon in einem Überschuß vorliegen. Besonders bevorzugt, weil dadurch eine besonders hohe Empfindlichkeit erzielt wird, ist es, wenn Konjugat 1 in einem Überschuß zu Konjugat 2 vorliegt. Als besonders günstig hat sich ein sechs- bis zehnfacher Überschuß von Konjugat 1 gegenüber Konjugat 2 herausgestellt.

Für den Fall, daß die Probe mit einem zusätzlichen Elutionsmittel durch das erfindungsgemäße Element in die Nachweiszone transportiert werden soll, hat sich ein Kit als günstig erwiesen, der aus dem erfindungsgemäßen Analysenelement und einem entsprechenden Elutionsmittel besteht. Das Elutionsmittel kann hierbei Wasser oder eine wässrige Lösung, bevorzugt eine Pufferlösung, sein, wobei sich das Elutionsmittel in einem entsprechenden Behältnis befindet. Dieses Behältnis kann beispielsweise eine Tropfflasche sein, um die Flüssigkeit auf die Elutionsmittelaufgabezone aufzugeben. Es kann aber auch beispielsweise ein Becher sein, der unbenutzt mit einem Deckel verschlossen ist, zur Durchführung des Bestimmungsverfahrens aber entdeckelt werden kann, und ein erfindungsgemäßes Element so in den die Elutionsmittelflüssigkeit enthaltenden Becher gestellt werden kann, daß Elutionsmittel über die Elutionsmittelaufgabezone aufgenommen wird und die verschiedenen Zonen bis in die Nachweiszone durchwandert.

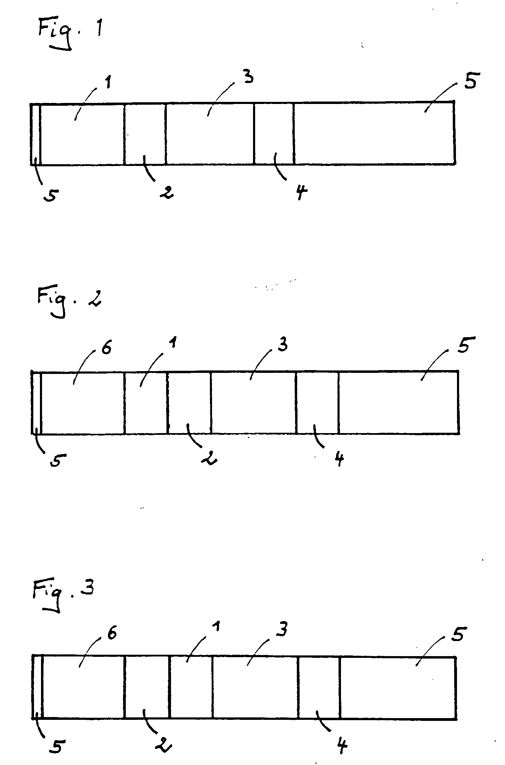

In Fig. 1 - 4 werden vier verschiedene mögliche Ausführungsformen eines erfindungsgemäßen Elementes in Aufsicht gezeigt.

Eine besonders bevorzugte Ausführungsform eines erfindungsgemäßen Elementes ist in Figur 1 dargestellt. Auf einer steifen, inerten Trägerfolie (5) sind Matrixmaterialien (1- 4) nebeneinander so befestigt, daß sie mit ihren Stirnflächen aneinanderstoßen bzw. leicht überlappen. Die Matrixmaterialien (1- 4) repräsentieren die Testzonen des erfindungsgemäßen Analysenelementes. Sie bestehen bevorzugt jeweils aus verschiedenen saugfähigen Materialien (Papieren, Vliesen, porösen Kunststoffschichten und dergleichen), wobei der Fluidkontakt an den Stoßkanten durch ausreichend dichtes Aneinanderfügen der Schichten realisiert ist. In einer alternativen Ausführungsform ist es jedoch natürlich auch möglich, daß mehrere benachbarte Zonen aus dem gleichen Material einstückig oder mehrstückig hergestellt sind. Insgesamt bilden die Testzonen eine Flüssigkeitstransportstrecke, die von der Probenauftragszone (1) über die Konjugatzone (2), die Konjugat 1 und Konjugat 2 enthält, und die Zone mit immobilisiertem Analyt oder Analytanalogon (3), die immobilisierten Analyt oder Analytanalogon enthält, in die Nachweiszone (4) führt. In der Konjugatzone (2) können die Konjugate 1 und 2 nach vorheriger Mischung entsprechender Lösungen oder Suspensionen gemeinsam aufgebracht sein. Die Konjugatzone (2) kann auch erst mit einem Konjugat imprägniert und dann mit dem anderen Konjugat nachimprägniert sein. Oder die Konjugatezone (2) kann zwei übereinanderliegende, gleiche oder verschiedene, Matrixmaterialien enthalten, die jeweils ein anderes Konjugat tragen.

Im vorliegenden Fall handelt es sich um ein Analysenelement, bei dem entweder soviel flüssige Probe aufgegeben wird, daß das Flüssigkeitsvolumen ausreicht, alle Matrixmaterialien inklusive Nachweiszone (4) mit Flüssigkeit zu versorgen, oder bei dem zuerst Probe auf die Probenauftragszone (1) gegeben wird, die dann anschließend durch ein spezielles Elutionsmittel, das ebenfalls auf die Probenauftragszone (1) aufgegeben wird, durch das erfindungsgemäße Element transportiert wird. Im Falle einer Analyt enthaltenden Probe wird beim Transport des Analyts mit Flüssigkeit durch die verschiedenen Zonen (1- 4) ab der Konjugatzone (2) ein Nachweiskomplex aus Analyt und Konjugaten 1 und 2 gebildet. Dieser Komplex durchwandert die Zone mit immobilisiertem Analyt oder Analytanalogon (3) und gelangt in die Nachweiszone (4), wo beispielsweise eine als Markierung 2 dienende Goldmarkierung mit dem Auge als rote Färbung erkennbar ist. Bei Probe, die keinen Analyt enthält, werden beim Flüssigkeitstransport durch die Zonen (1- 4) des erfindungsgemäßen Elementes die Konjugate aus der Konjugatzone (2) in die Zone mit immobilisiertem Analyt oder Analytanalogon (3) transportiert, wo die Konjugatmischung an immobilisiertem Analyt oder Analytanalogon gebunden wird. Visuell erkennbare Markierung 2 wird in diesem Fall nicht in die Nachweiszone (4) gelangen. Es wird dort keine Verfärbung erkennbar sein.

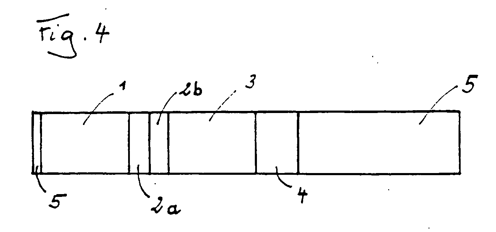

In Fig. 2 und 3 sind erfindungsgemäße Elemente dargestellt, die der Probenauftragszone (1) eine Elutionsmittelaufgabezone (6) vorgeschaltet haben. Bei der Anwendung solcher Elemente wird Probe zunächst auf die Probenauftragszone (1) gegeben. Anschließend wird soviel Elutionsmittel auf die Elutionsmittelaufgabezone (6) gegeben, daß Analyt in die Konjugatzone (2) transportiert wird, wo eine Komplexbildung mit Konjugaten 1 und 2 erfolgen kann, und daß der gebildete Komplex über die Zone (3) mit immobilisiertem Analyt oder Analytanalogon in die Nachweiszone (4) gelangt, wo dann der Nachweis des Analyts erfolgt.

Die Elemente der Fig. 2 oder 3 können allerdings nach dem Probenauftrag auch mit der Elutionsmittelaufgabezone (6) in soviel Elutionsmittel gestellt werden, daß sich die Probenauftragszone (1) bei einem Element gemäß Fig. 2 beziehungsweise die Konjugatzone (2) bei einem Element gemäß Fig.3 über dem Flüssigkeitsspiegel des Elutionsmittels befinden.

Während bei Fig. 2 die Reihenfolge von Zonen (1- 4) die gleiche ist wie bei dem Element gemäß Fig. 1, ist die Reihenfolge von Probenauftragszone (1) und Konjugatzone (2) in dem Element gemäß Fig. 3 vertauscht. Hier kommt Elutionsmittel zuerst mit Konjugaten 1 und 2 in Kontakt und inkubiert diese vor, bevor der Analyt kontaktiert und gebunden wird.

Fig. 4 zeigt ein erfindungsgemäßes Element, das zwei nebeneinander angeordnete Zonen 2a und 2b enthält, die jeweils ein anderes der beiden Konjugate 1 und 2 tragen. Die Konjugate können sich dabei auf dem gleichen, ein- oder zweistückig angeordneten Matrixmaterial befinden oder sie können auf verschiedenartigen Matrixmaterialien vorhanden sein. Die Reihenfolge der Zonen (1), (3) und (4) untereinander und in bezug auf die Konjugatzone, die in die Teilbereiche 2a und 2b aufgeteilt ist, ist identisch mit der in Fig. 1 gezeigten. Die Funktion des Elementes gemäß Fig. 4 entspricht deshalb, mit Ausnahme der nacheinander erfolgenden Lösung der Konjugate 1 und 2, der für das Element gemäß Fig. 1 beschriebenen.

Die Erfindung wird durch das nachfolgende Beispiel noch näher erläutert.

### Beispiel 1

### Bestimmung von Benzodiazepin mit einem Element gemäß Figur 1

### A. Herstellung von Konjugat aus Gold und monoklonalem Antikörper gegen Digoxin

Es werden zwei Konjugate hergestellt. Konjugat A enthält Goldpartikel mit etwa 40 nm Größe, beladen bei einer Antikörperkonzentration von 2 mg/l. Konjugat B enthält Goldpartikel mit ca. 20 nm Größe, beladen bei einer Antikörperkonzentration von 10 mg/l.

Goldsol mit einem mittleren Partikeldurchmesser von ca. 40 nm bzw. ca. 20 nm wurde nach der Methode von Frens (Frens, G., "Preparation of gold dispersions of varying particle size: Controlled nucleation for the regulation of the particle size in monodisperse gold suspensions" in Nature: Physical Science 241 (1973), 20-22) durch Reduktion einer 0,01-gewichtsprozentigen Tetrachlorgoldsäurelösung unter Kochen mittels Trinatriumcitrat hergestellt.

Die Antikörper-Goldkonjugat-Herstellung erfolgte in Anlehnung an die Methode von Roth, J. "The colloidal gold marker system for light and electrone microscopic cytochemistry" in Bullock, G. R. and Petrusz, P., eds., "Techniques in Immunocytochemistry", Vol. 2. New York, Academic Press, 1983, 216-284.

Nach dem Abkühlen der wie zuvor beschriebenen Goldsol-Lösung auf Raumtemperatur wurde der pH-Wert des Goldsols mit 0,2M Kaliumcarbonat-Lösung auf pH 8,0 eingestellt. Zum Goldsol wurde eine dialysierte Lösung eines monoklonalen IgG-Antikörpers gegen Digoxin (Bezugsquelle: Roche Diagnostics GmbH, Mannheim, Deutschland) zugegeben. Das Volumenverhältnis IgG-Lösung zu kolloidaler Goldlösung war dabei 1: 10. Nach 30 Minuten Rühren bei Raumtemperatur wurde das Goldkonjugat durch Zugabe einer hochkonzentrierten Rinderserumalbumin-Lösung (Endkonzentration in der Konjugatlösung: 1 mg/ml) abgesättigt.

Das Goldkonjujgat wurde durch Ultrafiltration gegen einen 20 mM Tris-Puffer pH 8,0 auf eine optische Dichte von typischerweise 20 (Extinktion bei 525 nm und 1 cm Lichtweg) aufkonzentriert. Die Konjugatlösung wurde abschließend auf 100 µM Brij® und 0,05 Gewichtsprozent Natriumazid aufgestockt.

### B. Herstellung von Konjugat aus Digoxin und polyklonalem Antikörper gegen Benzodiazepin

Schafe werden mit 7-Chlor-3-[2-(N-Maleinimido)ethyl]oxy-1 -methyl-5-phenyl-1H-1,4-benzodiazepin-2(3H)-on, dessen Synthese aus Beispiel 3 von EP-A 0726275 bekannt ist, wie in Beispiel 1 von EP-A 0726275 immunisiert. 100 ml Serum eines entsprechend immunisierten Schafes, enthaltend ca. 6,5 g Protein, wurden zuerst mit 1,5 g Aerosil behandelt (1 Stunde bei Raumtemperatur) und abzentrifugiert. Der Überstand wurde abdekantiert und mit Ammoniumsulfat auf 1,9M eingestellt. Dabei wurde das IgG gefällt. Der Niederschlag wurde abzentrifugiert und der Überstand abgegossen. Der Niederschlag wurde in schwachem PBS-Puffer pH 7 aufgenommen und dialysiert. Das Dialysat wurde über 100 ml DEAE-Sephadex ff negativ aufgereinigt. Dabei blieben Verunreinigungen auf der Säule und das Immunglobulin lief durch. Der Durchlauf wurde aufgefangen (Detektion bei 280 nm) und umgepuffert in PBS-Puffer pH 7,4 für die Immunsorption. Ausbeute 3,5 g IgG in 90 ml Lösung.

3,5 g IgG in 90 ml aus der DEAE-Aufreinigung wurden über 50 ml Immunadsorber (Spherosil, an das ein Polyhapten aus Temazepam auf Kaninchen IgG, (siehe Beispiel 1D), gebunden ist) im Kreislauf gepumpt. Dabei banden die immunspezifischen IgGs und alle anderen Proteine liefen durch. Mit PBS-Puffer wurde nachgewaschen. Das gebundene IgG wurde noch mit zwei Säulenvolumen Kochsalz/Tween 20 und zwei Säulenvolumen 30mM Natriumchlorid gewaschen. Dann wurde stufenweise eluiert. Erste Stufe ist die Elution mit 3mM Salzsäure bei 4 bis 8 °C, zweite Stufe ist die Elution mit 1M Propionsäure bei 4 bis 8 °C und dritte Stufe die Elution mit 1M Propionsäure bei Raumtemperatur. Die Eluate wurden sofort gegen Eiswasser dialysiert. Die Eluate wurden weiter gegen 1 mM Essigsäure dialysiert und nach dem Aufkonzentrieren filtriert und lyophilisiert. Ausbeute etwa 300 mg polyklonale IgG-Antikörper gegen Benzodiazepin.

Die so hergestellten polyklonalen Antikörper wurden in Wasser gelöst, so daß eine Konzentration von 12 mg Antikörper pro ml eingestellt wurde. Es wurde mit 1,5 ml 1M Kaliumphosphatpuffer pH 8,3 auf 0,1M Kaliumphosphat umgepuffert und auf 10 mg Antikörper pro ml eingestellt. Digoxinsuccinimidester wurde in DMSO zu einer Konzentration von 10 mg/ml gelöst und ein 6facher molarer Überschuß des Digoxin-Derivates bei 4 °C in die Antikörper-Lösung pipettiert. Es wurde drei Stunden bei 4 °C reagieren gelassen. Anschließend erfolgte Dialyse jeweils gegen das 50fache Volumen an 20mM Tris pH 8 und gegen das 50fache Volumen an 50mM Natriumchlorid über mindestens je vier Stunden.

### C. Herstellung von polymerisiertem Streptavidin

Polymerisiertes Streptavidin wurde, wie in Beispielen 1c und 1d in EP-B 0331127 beschrieben, hergestellt.

### D. Herstellung von biotinyliertem Temazepam-Polyhapten

1,2 g unspezifisches, lyophilisiertes polyklonales Kaninchen-IgG wurde in 40 ml Kaliumphosphat-Puffer pH 8,5 gelöst und nach Zentrifugation der klare Überstand abdekantiert. Der Überstand enthielt 950 mg Protein. 12.4 mg S-Acetylthiopropionsäuresuccinimidylester, in 1,24 ml DMSO gelöst, wurden zur Kaninchen-IgG-Lösung gegeben und zwei Stunden bei Raumtemperatur gerührt. Die Reaktion wurde durch Zugabe von 0,5 ml 1 M Lysin-Lösung gestoppt und gegen Triethylammoniumcitrat/EDTA-Lösung pH 6,5 dialysiert.

Das aktivierte Kaninchen-Immunglobulin wurde nach der Dialyse mit 1 ml 1M Ammoniumhydroxid-Lösung pro 25 ml Immunglobulin-Lösung bei pH 6,5 zwei Stunden lang bei 4 °C deacetyliert.

Für 950 mg deacetyliertes aktiviertes Kaninchen-IgG in 50 ml Puffer wurden 10,8 mg Temazepam (Herstellung gemäß Beispiel 3 in EP-A 0726275) in 1,08 ml DMSO gelöst und in die deacetylierte aktivierte Kaninchen-Immunglobulin-Lösung gegeben. Nach zwei Stunden bei 4 °C wurde die Reaktion mit 2 ml 0,1 M Cystein abgestoppt und die Lösung 30 Minuten mit 2 ml 0,5M Jodacetamid inkubiert und anschließend 30 Minuten bei Raumtemperatur gegen Triäthanolaminpuffer pH 8,5 dialysiert.

Zu 950 mg des so hergestellten Temazepam-Polyhaptens wurden 24 mg Biotinsuccinimidylester, gelöst in 2,4 ml DMSO, zugegeben und zwei Stunden bei Raumtemperatur gerührt. Anschließend erfolgte eine intensive Dialyse gegen schwache Essigsäure (10 bis 1 mM). Nach der Dialyse wurde das Dialysat mit 1M Natriumacetat pH 4,2 auf 20mM Natriumacetat pH 4,2 eingestellt und über Trisacryl-Carboxymethylcellulose säulenchromatographisch aufgereinigt.

### E. Herstellung von Analysenelementen gemäß Figur 1

Es wurden 2 erfindungsgemäße Analyseelemente mit unterschiedlichen Konjugatzonen (2) hergestellt.

Auf Trägerfolie (5) von 5 mm Breite wurden die folgenden Zonen gemäß Figur 1 geklebt.

Probenauftragszone (1): Polyestervlies der Firma Binzer, Hatzfeld, Deutschland. Ein reines Polyestervlies, das mit 10 % Kuralon® verfestigt ist, eine Dicke von 1,0 bis 1,2 mm aufweist und eine Saugkapazität von 1800 ml/m² besitzt.

Konjugatzone (2): Je ein Mischvlies aus 80 Teilen Polyester und 20 Teilen Zellwolle, verfestigt mit 20 Teilen Kuralon®, in einer Dicke von 0,32 mm und mit einer Saugkapazität von 500 ml/m², wurde mit einer der folgenden Lösungen getränkt und getrocknet:
- Tränklösung A
   Eine Mischung von jeweils 1 ml des in A. hergestellten Goldkonjugats A, verdünnt in Hepes-Puffer (200mM, pH 7,5) auf eine Antikörperkonzentration von 0,3nmol/ml, und 1 ml des in B. hergestellten Digoxinkonjugats, verdünnt in Hepes-Puffer (200mM, pH 7,5) auf eine Digoxin-Konzentration von 2 nmol/ml, wird eine Stunde bei Raumtemperatur inkubiert. Anschließend erfolgt die Tränkung in das Vlies.
- Tränklösung B
   Eine Mischung von jeweils 1 ml des in A. hergestellten Goldkonjugats B, verdünnt in Hepes-Puffer (200mM, pH 7,5) auf eine Antikörperkonzentration von 1,4 nmol/ml und 1 ml des in B. hergestellten Digoxin-Konjugats, verdünnt in Hepes-Puffer (200mM, pH 7,5) auf eine Digoxin-Konzentration von 2 nmol/ml wird eine Stunde bei Raumtemperatur inkubiert. Anschließend erfolgt die Tränkung in das Vlies.

Zone mit immobilisiertem Analyt oder Analytanalogon (3): Ein Vlies aus 100 % Linters, verfestigt mit zwei Gewichtsprozent Etadurin mit einer Dicke von 0,41 mm und einer Saugkapazität von 386 ml/m², wird mit einer Lösung von 200 mg/l des in C hergestellten polymerisierten Streptavidins in 50 mmol/l Natriumphosphat pH 8,0 getränkt und anschließend getrocknet. Das vorgetränkte Vlies wird anschließend mit einer Lösung von 300 mg/l des in D. hergestellten biotinylierten Temazepam-Polyhaptens in 50 mmol/l Natriumphosphat pH 8,0 nochmals getränkt und getrocknet.

Nachweiszone (4): Es wird ein Vlies aus 100 % Linters, verfestigt mit zwei Gewichtsprozent Etadurin, mit einer Dicke von 0,35 mm und einer Saugkapazität von 372 ml/m², eingesetzt.

### F. Testdurchführung

Zur Bestimmung von Benzodiazepin mit einem in E. hergestellten Element wird der Teststreifen für ca. 5 Sekunden in die zu untersuchende Flüssigkeit getaucht, so daß sich die Probenauftragszone (1) etwa zu drei Viertel unterhalb des Flüssigkeitsspiegels befindet. Danach wird das Element auf einer nicht saugenden Unterlage waagerecht abgelegt. Wenn die Flüssigkeitsfront die Nachweiszone (4) komplett benetzt hat (bei wässrigen Lösungen in der Regel nach maximal zwei Minuten), zeigt eine Rosaverfärbung die Anwesenheit von Benzodiazepinen in der zu untersuchenden Probe an. Bei Abwesenheit von Substanzen, die der Digoxin-markierte Antikörper erkennt, bleibt das Nachweisfeld weiß. Die Intensität der Rosafärbung korreliert mit der Analytkonzentration. Eine Farbtafel vereinfacht die Zuordnung:
Farbfeld (FF) 0: negativ, kein Analyt
Farbfeld (FF) 1: positiv, helles Rosa
Farbfeld (FF) 2: stark positiv, kräftige Rotfärbung

### Ergebnistabelle

| | Wässrige Lösung mit Bromazepam | | Urin mit Bromazepam | | |
|---|---|---|---|---|---|
| | 0ng/ml | 100ng/ml | 0ng/ml | 50 ng/ml | 100ng/ml |
| Gold-Konjugat A | FF 0 | FF 1-2 | FF 0 | FF 0-1 | FF 1-2 |
| Gold-Konjugat B | FF 0 | FF 2 | - | - | - |

## Patentansprüche

1. Element zur Bestimmung eines Analyts in einer Flüssigkeit mittels einer spezifischen Bindungsreaktion zweier bioaffiner Bindungspartner
enthaltend in oder auf Material, das einen Flüssigkeitstransport zwischen Zonen ermöglicht, eine Probenauftragszone (1) und eine hierzu flußabwärts gelegene Nachweiszone (4) sowie zwischen Probenauftragszone (1) und Nachweiszone (4) eine Zone (3) mit immobilisiertem Analyt oder Analytanalogon
und flußaufwärts der Zone (3) mit immobilisiertem Analyt oder Analytanalogon durch Flüssigkeit ablösbar imprägniertes Konjugat 1 aus einem mit dem zu bestimmenden Analyt zu einer spezifischen Bindungsreaktion fähigen bioaffinen Bindungspartner 1 und einer detektierbaren Markierung 1,
**dadurch gekennzeichnet, dass**
die detektierbare Markierung 1 ein niedermolekulares organisches Molekül ist, das ein Molekulargewicht kleiner als 1500 hat und das als Hapten von einem entsprechenden Antikörper in einer spezifischen Bindungsreaktion gebunden und so detektiert werden kann, und dass flußaufwärts der Zone (3) mit immobilisiertem Analyt oder Analytanalogon ebenfalls durch Flüssigkeit ablösbares, universelles Konjugat 2 aus einem mit der detektierbaren Markierung 1 zu einer spezifischen Bindungsreaktion fähigen bioaffinen Bindungspartner 2 und einer visuell erkennbaren Markierung 2 vorhanden ist, wobei die Markierung 2 ohne weitere Handhabungsschritte mit dem Auge durch ihre Farbe erkennbar ist,
und dass Konjugat 1 in einem Überschuss zu Konjugat 2 vorliegt.

2. Element gemäß Anspruch 1, **dadurch gekennzeichnet, daß** die detektierbare Markierung 1 Digoxygenin oder Digoxin ist.

3. Element gemäß Anspruch 1 oder 2, **dadurch gekennzeichnet, daß** der bioaffine Bindungspartner 2 ein Antikörper gegen Digoxigenin oder Digoxin ist.

4. Element gemäß einem der Ansprüche 1 bis 3, **dadurch gekennzeichnet, daß** als visuell erkennbare Markierung 2 Metall- oder Latexpartikel verwendet werden.

5. Element gemäß Anspruch 4, **dadurch gekennzeichnet, daß** als visuell erkennbare Markierung 2 Goldpartikel verwendet werden.

6. Element gemäß einem der Ansprüche 1 bis 5, **dadurch gekennzeichnet, daß** sich flußaufwärts der Probenauftragszone (1) eine Elutionsmittelaufgabezone (6) befindet.

7. Element gemäß Anspruch 1, **dadurch gekennzeichnet, daß** sich Konjugat 1 und Konjugat 2 zwischen Probenauftragszone (1) und Zone (3) mit immobilisiertem Analyt oder Analytanalogon befinden.

8. Element gemäß Anspruch 6, **dadurch gekennzeichnet, daß** sich Konjugat 1 und Konjugat 2 zwischen Elutionsmittelaufgabezone (6) und Probenauftragszone (1) befinden.

9. Element gemäß Anspruch 1, **dadurch gekennzeichnet, daß** sich Konjugat 1 und Konjugat 2 in der Probenauftragszone (1) befinden.

10. Verfahren zur Bestimmung eines Analyts mittels eines Elementes gemäß Ansprüchen 1 bis 9,
**dadurch gekennzeichnet, dass**
die Probenauftragszone (1) mit Analyt in Kontakt gebracht wird, der Analyt mit Flüssigkeit in Richtung Nachweiszone (4) bewegt wird, in dieser Flüssigkeit befindlicher Analyt mit Konjugaten 1 und 2 zu einem Nachweiskomplex reagiert,
der Nachweiskomplex mit Flüssigkeit in die Nachweiszone (4) transportiert und dort bestimmt wird,
wobei Konjugat 1 in einem Überschuss zu Konjugat 2 vorliegt, und
wobei Konjugat 1 einen mit dem zu bestimmenden Analyt zu einer spezifischen Bindungsreaktion fähigen bioaffinen Bindungspartner 1 und eine detektierbare Markierung 1 aufweist, die ein niedermolekulares organisches Molekül ist, das ein Molekulargewicht kleiner als 1500 hat und das als Hapten von einem entsprechenden Antikörper in einer spezifischen Bindungsreaktion gebunden und so detektiert werden kann, und
Konjugat 2 einen mit der detektierbaren Markierung 1 zu einer spezifischen Bindungsreaktion fähigen bioaffinen Bindungspartner 2 und eine visuell erkennbare Markierung 2 aufweist, wobei die Markierung 2 ohne weitere Handhabungsschritte mit dem Auge durch ihre Farbe erkennbar ist,und
Konjugat 2 mittels des mit der detektierbaren Markierung 1 zu einer spezifischen Bindungsreaktion fähigen Bindungspartners 2 spezifisch an die detektierbare Markierung 1 des Konjugats 1 bindet.

11. Verfahren gemäß Anspruch 10, **dadurch gekennzeichnet, daß** die Flüssigkeit eine Probenflüssigkeit ist, mit der der Analyt auf das Element gebracht wird.

12. Verfahren gemäß Anspruch 10, **dadurch gekennzeichnet, daß** zum Bewegen des Analyts zusätzliches Elutionsmittel auf die Elutionsmittelaufgabezone (6) gemäß Anspruch 6 gegeben wird.

13. Verwendung eines Elementes gemäß Ansprüchen 1 bis 9 zur Bestimmung eines Analyts.

14. Kit zur Bestimmung eines Analyts enthaltend ein Element gemäß Ansprüchen 1 bis 9 und Elutionsmittel.

## Claims

1. Element for the determination of an analyte in a liquid by means of a specific binding reaction of two bioaffine binding partners
containing in or on material which enables liquid transport between zones, a sample application zone (1) and a detection zone (4) located downstream thereof as well as a zone (3) containing immobilized analyte or analyte analogue between the sample application zone (1) and detection zone (4) and an impregnated conjugate 1 located upstream of the zone (3) containing immobilized analyte or analyte analogue that can be detached by liquid and is composed of a bioaffine binding partner 1 capable of a specific binding reaction with the analyte to be determined and a detectable label 1,
**characterized in that**
the detectable label 1 is a low-molecular organic molecule which has a molecular weight of less than 1500 and which, as a hapten, can be bound by a corresponding antibody in a specific binding reaction and can thus be detected, and that a universal conjugate 2 which can also be detached by liquid is present upstream of the zone (3) containing immobilized analyte or analyte analogue and is composed of a bioaffine binding partner 2 capable of a specific binding reaction with the detectable label 1 and a visually detectable label 2 wherein the label 2 is visually detectable without further handling steps by means of its colour and conjugate I is present in an excess relative to conjugate 2.

2. Element according to claim 1, **characterized in that** the detectable label 1 is digoxigenin or digoxin.

3. Element according to claim 1 or 2, **characterized in that** the bioaffine binding partner 2 is an antibody to digoxigenin or digoxin.

4. Element according to one of the claims 1 to 3, **characterized in that** metal or latex particles are used as a visually detectable label 2.

5. Element according to claim 4, **characterized in that** gold particles are used as the visually detectable label 2.

6. Element according to one of the claims 1 to 5, **characterized in that** an elution agent application zone (6) is located upstream of the sample application zone (1).

7. Element according to claim 1, **characterized in that** conjugate 1 and conjugate 2 are located between the sample application zone (1) and zone (3) containing immobilized analyte or analyte analogue.

8. Element according to claim 6, **characterized in that** conjugate 1 and conjugate 2 are located between the elution agent application zone (6) and the sample application zone (1).

9. Element according to claim 1, **characterized in that** conjugate 1 and conjugate 2 are located in the sample application zone (1).

10. Method for the determination of an analyte using an element according to claims 1 to 9, **characterized in that** the sample application zone (1) is contacted with analyte, the analyte is moved by the liquid towards the detection zone (4), analyte present in this liquid reacts with conjugates 1 and 2 to form a detection complex, the detection complex is transported with liquid into the detection zone (4) and is determined there, **characterized in that** conjugate 1 is present in an excess relative to conjugate 2 and wherein conjugate 1 has a bioaffine binding partner 1 capable of a specific binding reaction with the analyte to be determined and a detectable label 1 which is a low-molecular organic molecule which has a molecular weight of less than 1500 and which, as a hapten, is bound by a corresponding antibody in a specific binding reaction and can thus be detected, and
conjugate 2 has a bioaffine binding partner 2 capable of a specific binding reaction with the detectable label 1 and a visually detectable label 2, wherein the label 2 can be visually detected without further handling steps by means of its colour and
conjugate 2 binds specifically to the detectable label 1 of the conjugate 1 by means of the binding partner 2 capable of a specific binding reaction with the detectable label 1.

11. Method according to claim 10, **characterized in that** the liquid is a sample liquid which is used to bring the analyte onto the element.

12. Method according to claim 10, **characterized in that** an additional elution agent is added to the elution agent application zone (6) as claimed in claim 6 to move the analyte.

13. Use of an element according to claims 1 to 9 to determine an analyte.

14. Kit for determining an analyte containing an element according to claims 1 to 9 and an elution agent.

## Revendications

1. Élément de détermination d'un analyte dans un liquide au moyen d'une réaction de liaison spécifique entre deux partenaires de liaison qui présentent une bioaffinité,
contenant, dans ou sur une substance qui permet un transport de liquide entre zones,
une zone d'application d'échantillon (1) et une zone de détection (4) située en aval de la précédente, ainsi qu'une zone (3) comprenant un analyte ou analogue d'analyte immobilisé, située entre la zone d'application d'échantillon (1) et la zone de détection (4),
et en amont de la zone (3) comprenant un analyte ou analogue d'analyte immobilisé, un conjugué 1 imprégné de manière déplaçable par un liquide, constitué d'un partenaire de liaison 1 présentant une bioaffinité, capable de se lier en une réaction de liaison spécifique avec l'analyte à déterminer, et un marqueur détectable 1,
**caractérisé en ce que**
le marqueur détectable 1 est une molécule organique de faible poids moléculaire, inférieur à 1500, qui est capable de se lier en tant qu'haptène avec un anticorps approprié dans une réaction de liaison spécifique et d'être ainsi détectée,
et **en ce que** en amont de la zone (3) comprenant un analyte ou analogue d'analyte immobilisé, se trouve un conjugué 2 universel, également déplaçable par un liquide, constitué d'un partenaire de liaison 2 présentant une bioaffinité, capable de se lier en une réaction de liaison spécifique avec le marqueur détectable 1, et un marqueur 2 détectable visuellement, ce marqueur 2 étant détectable à l'oeil nu par sa coloration sans autre étape de traitement,
et **en ce que** le conjugué 1 est présent en excès par rapport au conjugué 2.

2. Élément selon la revendication 1, **caractérisé en ce que** le marqueur détectable 1 est la digoxygénine ou la digoxine.

3. Élément selon la revendication 1 ou 2, **caractérisé en ce que** le partenaire de liaison 2 présentant une bioaffinité est un anticorps dirigé contre la digoxygénine ou la digoxine.

4. Élément selon l'une quelconque des revendications 1 à 3, **caractérisé en ce que** comme marqueur 2 détectable visuellement, on utilise des particules de métal ou de latex.

5. Élément selon la revendication 4, **caractérisé en ce que** comme marqueur 2 détectable visuellement on utilise des particules d'or.

6. Élément selon l'une quelconque des revendications 1 à 5, **caractérisé en ce qu'**une zone d'introduction d'éluant (6) se trouve en amont de la zone d'application d'échantillon (1).

7. Élément selon la revendication 1, **caractérisé en ce que** le conjugué 1 et le conjugué 2 se trouvent entre la zone d'application d'échantillon (1) et la zone (3) comprenant un analyte ou analogue d'analyte immobilisé.

8. Élément selon la revendication 6, **caractérisé en ce que** le conjugué 1 et le conjugué 2 se trouvent entre la zone d'introduction d'éluant (6) et la zone d'application d'échantillon (1).

9. Élément selon la revendication 1, **caractérisé en ce que** le conjugué 1 et le conjugué 2 se trouvent dans la zone d'application d'échantillon (1).

10. Procédé de détermination d'un analyte au moyen d'un élément selon les revendications 1 à 9, **caractérisé en ce que**
la zone d'application d'échantillon (1) est mise en contact avec l'analyte, l'analyte est transporté avec le liquide en direction de la zone de détection (4), l'analyte se trouvant dans ce liquide réagit avec les conjugués 1 et 2 pour donner un complexe de détection,
le complexe de détection est transporté avec le liquide dans la zone de détection (4) et y est déterminé,
le conjugué 1 étant présent en excès par rapport au conjugué 2, et
le conjugué 1 comprenant un partenaire de liaison 1 qui présente une bioaffinité et est capable de se lier en une réaction de liaison spécifique avec l'analyte à déterminer, et un marqueur détectable 1, ce dernier étant une molécule organique de faible poids moléculaire, inférieur à 1500, qui est capable de se lier en tant qu'haptène avec un anticorps approprié dans une réaction de liaison spécifique et d'être ainsi détectée, et
le conjugué 2 présente un partenaire de liaison 2 qui présente une bioaffinité et est capable de se lier en une réaction de liaison spécifique avec le marqueur détectable 1, et un marqueur 2 détectable visuellement, ce marqueur 2 étant détectable à l'oeil nu par sa coloration sans autre étape de traitement, et
le conjugué 2 forme une liaison spécifique avec le marqueur 1 détectable du conjugué 1 au moyen du partenaire de liaison 2 capable de se lier en une réaction de liaison spécifique avec le marqueur détectable 1.

11. Procédé selon la revendication 10, **caractérisé en ce que** le liquide est un liquide échantillon, avec lequel l'analyte est déposé sur l'élément.

12. Procédé selon la revendication 10, **caractérisé en ce que**, pour déplacer l'analyte, un éluant supplémentaire est appliqué sur la zone d'introduction d'éluant (6) selon la revendication 6.

13. Utilisation d'un élément selon les revendications 1 à 9 pour la détermination d'un analyte.

14. Kit de détermination d'un analyte contenant un élément selon les revendications 1 à 9 et un éluant.
